# EUROPEAN PATENT SPECIFICATION

(11) **EP 2 979 709 B1**
(45) Date of publication and mention of the grant of the patent: **02.08.2017**
(21) Application number: 15183284.7
(22) Date of filing: 13.10.2011
(51) Int. Cl.: A61L 15/28, A61L 27/20, A61L 31/04, C08B 37/00

(54) **THREADS OF CROSS-LINKED HYALURONIC ACID AND USE THEREOF**
STRÄNGE AUS VERNETZTER HYALURONSÄURE UND VERWENDUNG DAVON
FILS D'ACIDE HYALURONIQUE RÉTICULÉ ET UTILISATION DE CEUX-CI

(30) Priority: 20.10.2010 US 405179 P
(43) Date of publication of application: 03.02.2016
(62) Divisional of application: 11776962.0
(73) Proprietor: Allergan Holdings France S.A.S., 92400 Courbevoie (FR)
(72) Inventor: Horne, Kenneth N., Menlo Park, CA California 94025 (US); Shenoy, Vivek, Menlo Park, CA California 94025 (US); Jayakumar, Naveen, Menlo Park, CA California 94025 (US); Gurtner, Geoffrey C., Menlo Park, CA California 94025 (US)
(74) Representative: Hoffmann Eitle

(56) References cited:
- EP-A1- 1 217 008
- EP-A1- 1 640 026
- WO-A1-2008/056069
- WO-A1-2010/028025
- US-A1- 2004 192 643

## Description

### FIELD

This disclosure relates generally to threads of hyaluronic acid, methods of making such threads and uses thereof as set forth in the claims.

### BACKGROUND

Hyaluronic acid is a linear polysaccharide (*i.e.,* non-sulfated glycosaminoglycan) consisting of a repeated disaccharide unit of alternately bonded β-D-N-acetylglucosamine and β-D-glucuronic acid which can be depicted by the formula:
where n is the number of repeating units. Hyaluronic acid is sometimes referred to by the nomenclature (-4GlcUAβ1-3GlcNAcβ1-)ₙ) and is a chief component of the extracellular matrix found, for example, in connective, epithelial and neural tissue. Natural hyaluronic acid is highly biocompatible because of its lack of species and organ specificity and is often used as a biomaterial in tissue engineering and as a common ingredient in soft tissue augmentation products.

Natural hyaluronic acid has poor *in vivo* stability due to rapid enzymatic degradation and hydrolysis and, accordingly, various chemically modified forms of hyaluronic acid (*e.g.,* cross-linked forms, ionically modified forms, esterified forms, *etc.*) have been synthesized to address this problem. Currently, hyaluronic acid or cross-linked versions thereof are used in various gel forms, for example as soft tissue augmentation products, adhesion barriers, and the like.

However, issues exist with the use of gels of hyaluronic acid or its cross-linked versions. First, the force required to dispense gels of hyaluronic acid or its cross-linked versions is non-linear which cause an initial ejection of a "glob" of gel that many physicians report when using hyaluronic acid. Second, precisely dispensing hyaluronic gels to specific locations can be difficult because such gels have little mechanical strength. Further, the gel will occupy the space of least resistance which makes its use in many applications (e.g., treatment of fine wrinkles) problematic as the gel will often migrate into unintended spatial areas rendering the cosmetic procedure difficult and possibly even dangerous. Many common soft tissue augmentation products which are injected into the treatment site as a liquid or a gel, are capable of migration and/or causing unsightly "lumps" which are painful to treat. Furthermore, these soft tissue augmentation products are not recommended for use around the eyes as migration from the injection site can cause blindness, tissue necrosis, and in rare cases even stroke. Clinicians also find performing lip augmentations using these fillers time consuming, and patients find treatments in this area so painful that nerve blocks are routinely performed.

Accordingly, there is a need for new physical forms of hyaluronic acid or its cross-linked versions which can be dispensed uniformly to specific locations regardless of tissue resistance, and without the risk of migration. Furthermore, it would be beneficial to have threads which can withstand various types of sterilizing. Such new forms will have particular uses, for example, in aesthetic and surgical applications, drug delivery, wound therapy and wound dressing.

### SUMMARY

Processes for fabricating hyaluronic acid threads have been disclosed earlier. These processes involve cross-linking the hyaluronic acid during the drying process after the threads have been extruded. In the present disclosure, the cross-linking of hyaluronic acid occurs prior to extrusion and drying, although some cross-linking can continue during the drying step. In addition, the cross-linked hyaluronic acid may be dried and irradiated prior to being formulated into a hyaluronic acid gel for extrusion and drying.

The threads fabricated using the process described herein and set forth in the claims are contemplated to degrade slower as compared to threads fabricated by processes described earlier. In addition, the threads fabricated with cross-linked hyaluronic acid which has been irradiated prior to formulation of the hyaluronic acid gel are contemplated to degrade slower than threads that are irradiated after the threads have been extruded and dried.

Further, it has been discovered that using certain ratios of cross-linking agent and hyaluronic acid in the composition prior to extrusion provides a composition that may be substantially cross-linked. Therefore, in one aspect, there is provided an aqueous composition comprising hyaluronic acid and a cross-linking agent, wherein the ratio by weight of the hyaluronic acid and the cross-linking agent is from about 1:2 to about 10:1.

In another aspect, there is provided a sterilized cross-linked hyaluronic acid having a degradation rate comparable to or slightly faster than the degradation rate of cross-linked hyaluronic acid prior to sterilization.

In another aspect, there is provided a cross-linked hyaluronic acid thread, prepared by the process of:
a) combining hyaluronic acid and a cross-linking agent in an aqueous composition until the hyaluronic acid is substantially cross-linked to provide a substantially cross-linked composition;
b) extruding the substantially cross-linked composition to provide a wet thread; and
c) drying the wet thread to provide a cross-linked hyaluronic acid thread.

In another aspect, there is provided a non-therapeutic method of treating a wrinkle in a patient in need thereof, said method comprising:
1) inserting a thread provided herein into skin of the patient adjacent to or under the wrinkle; and
2) applying the thread adjacent to or under the wrinkle thereby treating the wrinkle.

In another aspect, there is provided a cross-linked hyaluronic acid thread, obtainable by the process as set forth in claims 1 to 9, for use in a method of providing facial contouring in a patient in need thereof, said method comprising:
1) inserting a thread provided herein into skin of the patient adjacent to or under a treatment location; and
2) applying the thread adjacent to or under the treatment location thereby providing facial contouring.

It another aspect, there is provided a kit of parts comprising a thread as provided herein and a delivery device, such as a needle.

### BRIEF DESCRIPTION OF THE DRAWINGS

The disclosure is best understood from the following detailed description when read in conjunction with the accompanying drawings. It is emphasized that, according to common practice, the various features of the drawings are not to-scale. On the contrary, the dimensions of the various features are arbitrarily expanded or reduced for clarity. Included in the drawings are the following figures:

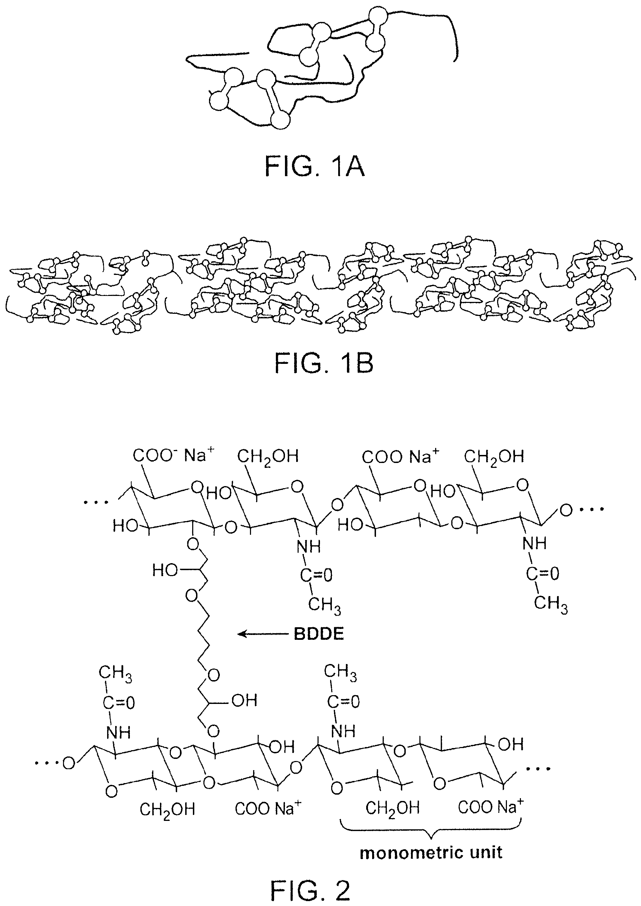

Fig. 1A shows a schematic illustration of cross-linked hyaluronic acids. The filament represents the HA and the ball and stick represent the cross-linking agent.
Fig. 1B shows a schematic illustration of a thread including cross-linked hyaluronic acids.
Fig. 2 shows a schematic of hyaluronic acid cross-linked with butanediol diglycidyl ether (BDDE).
Fig. 3 illustrates a thread attached to the proximal end of a needle, in its entirety (N = needle; T = thread).
Fig. 4 shows a needle attached to the thread (N = needle; T = thread). Fig. 4A illustrates a close-up view of a thread inserted into the inner-diameter of a needle; and Fig. 4B illustrates a close-up view of the proximal end of a solid needle with the thread overlapping the needle.

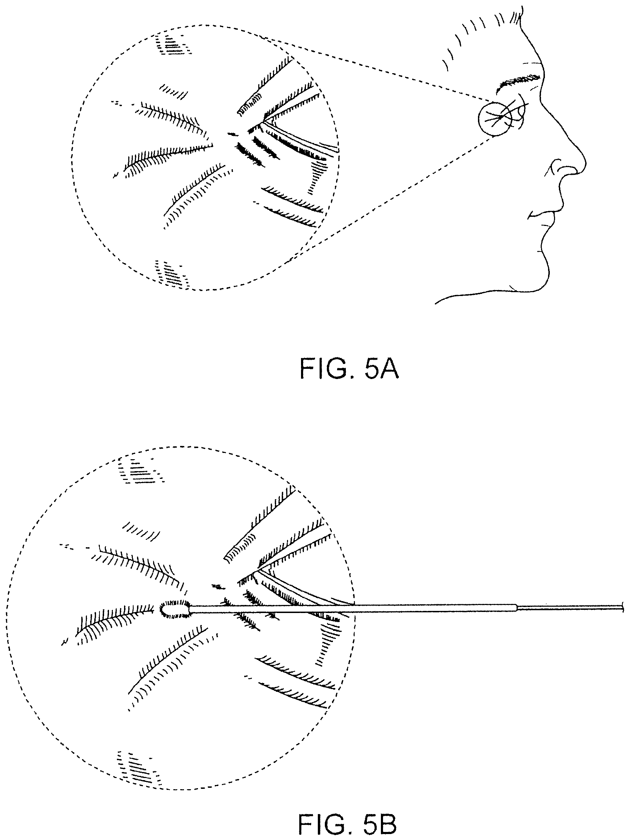

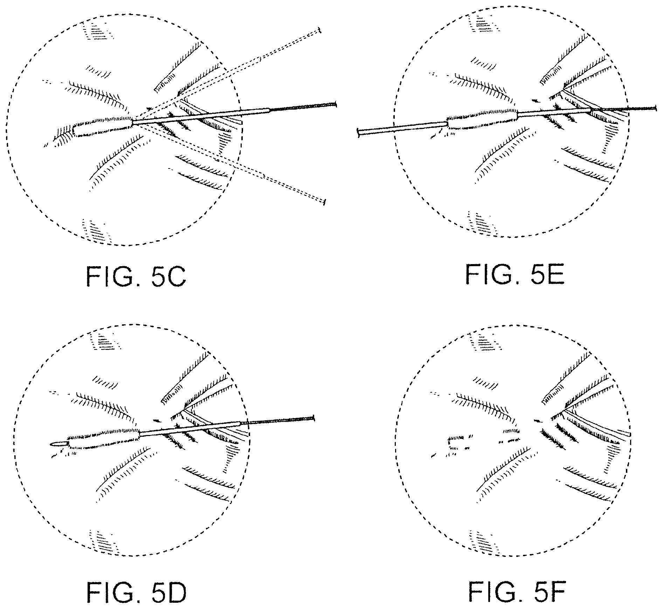

Fig. 5 shows treatment of a wrinkle. Fig. 5A illustrates a fine, facial wrinkle in the peri-orbital region of a human; Fig. 5B illustrates a needle and thread being inserted into the skin of the wrinkle at the medial margin; Fig. 5C illustrates the needle being adjusted to traverse beneath the wrinkle; Fig. 5D illustrates the needle exiting at the lateral margin of the wrinkle; Fig. 5E illustrates the needle having pulled the thread into the location it previously occupied beneath the wrinkle; and Fig. 5F illustrates the thread implanted beneath the wrinkle, with excess thread having been cut off.

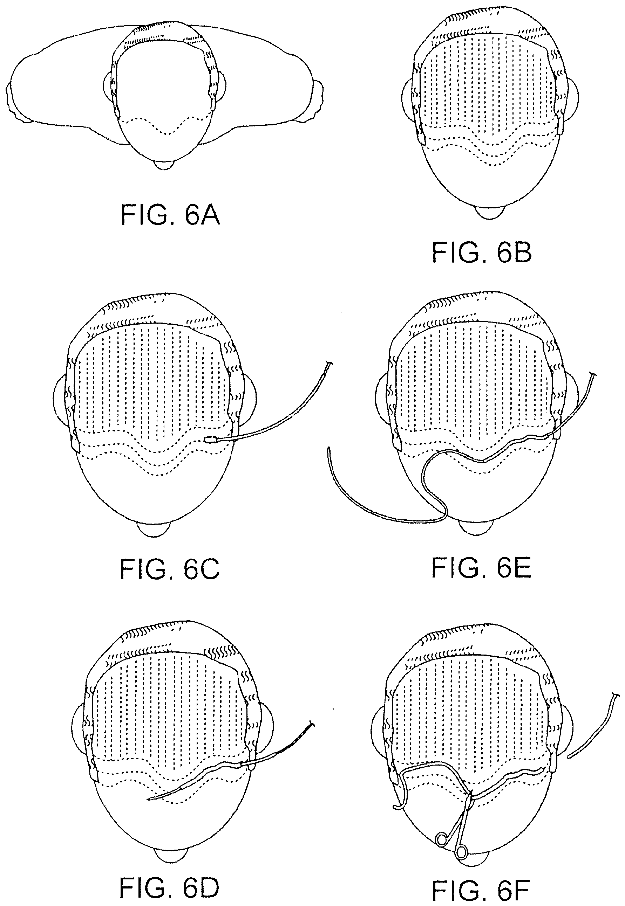

Fig. 6 shows treatment of baldness or hair regrowth. Fig. 6A illustrates a top-down view of a male with typical male-pattern baldness; Fig. 6B illustrates where hair re-growth is desired, taking hair-lines into consideration; Fig. 6C illustrates a curved needle with attached thread being inserted into one imaginary line where hair re-growth is desired; Fig. 6D illustrates the needle traversing the imaginary line, and exiting the skin; Fig. 6E illustrates the needle pulled through distally, pulling along the thread into the desired location; and Fig. 6F illustrates scissors being used to cut excess thread.
Fig. 7 shows treatment of a wrinkle. Fig. 7A illustrates a cross-sectional view of a fold or a wrinkle; Fig. 7B illustrates a thread implanted beneath a wrinkle that is not yet hydrated; and Fig. 7C illustrates a thread implanted beneath a wrinkle that is fully hydrated and has flattened the surface appearance of the wrinkle.

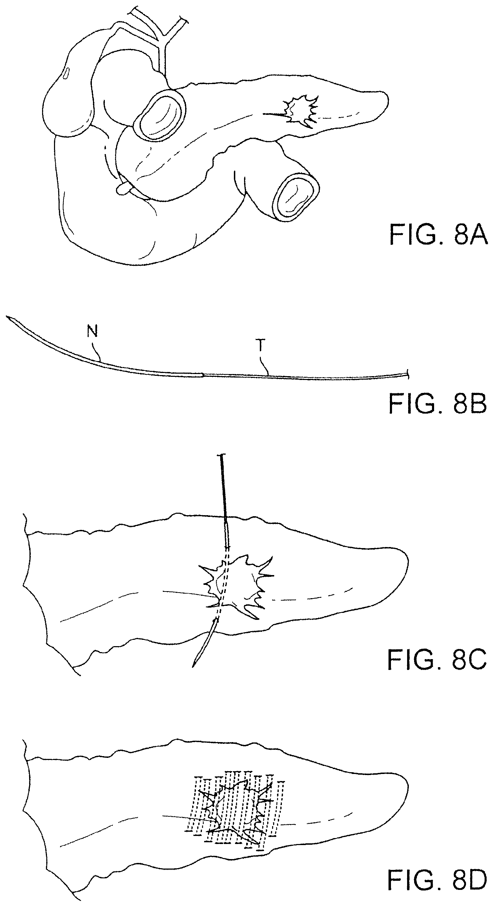

Fig. 8 shows treatment of a tumor. Fig. 8A illustrates a human pancreas with a tumor; Fig. 8B illustrates a curved needle with a thread attached thereto; Fig. 8C illustrates a curved needle traversing the tumor within the pancreas; and Fig. 8D illustrates the end-result of repeated implantations of thread.

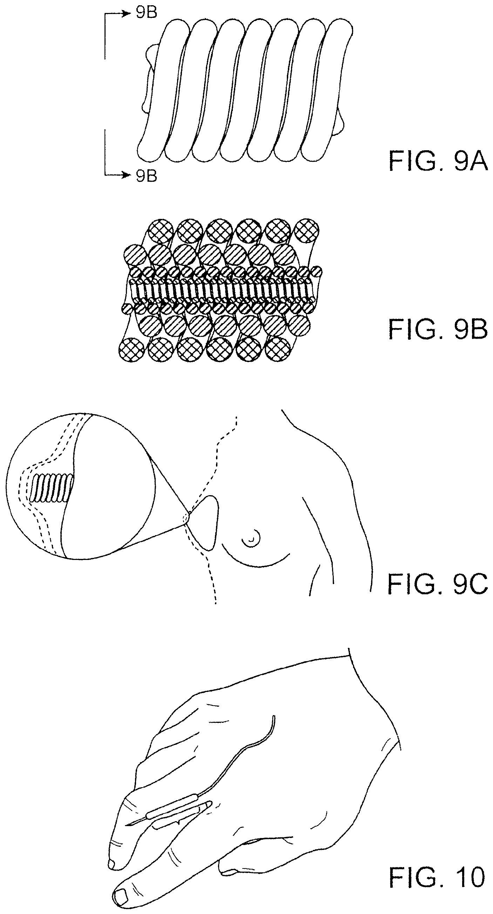

Fig. 9 shows a nipple reconstruction. Fig. 9A illustrates multiple layers of concentric coils of thread, shaped to represent a human nipple; Fig. 9B illustrates the implant of Fig. 9A in cross-section; and Fig. 9C illustrates how an implant of coiled thread would be used for nipple reconstruction.
Fig. 10 illustrates how a needle and thread could be used to place a thread in a specific, linear location to promote nerve or vessel regrowth in a specific line.
Fig. 11A shows placement of threads in a relatively parallel orientation for facial contouring in the tear trough (Thread 1, 2, 3, 4, 5, and 6). This figure also shows placement of the thread for facial contouring of the nasolabial fold (Thread 7 and 8). The threads may be placed in either the epidermis, dermis, subcutaneous layers, or combinations thereof.
Fig. 11B shows placement of threads is a cross-hatching manner for facial contouring in the tear trough (Thread 1, 2, 3, 4, 5, and 6). The threads may be placed in either the epidermis, dermis, subcutaneous layers, or combinations thereof.
Fig. 11C shows a cross-section of the dermal layers where threads have been delivered to various layers, including the epidermis, dermis, and subcutaneous layers in parallel planes.
Fig. 11D shows a three dimensional cross-section of the dermal layers in a hammock or cross-hatching manner. What may not be readily apparent from the figure is that the threads may be stratified across the various layers to enhance the contouring.

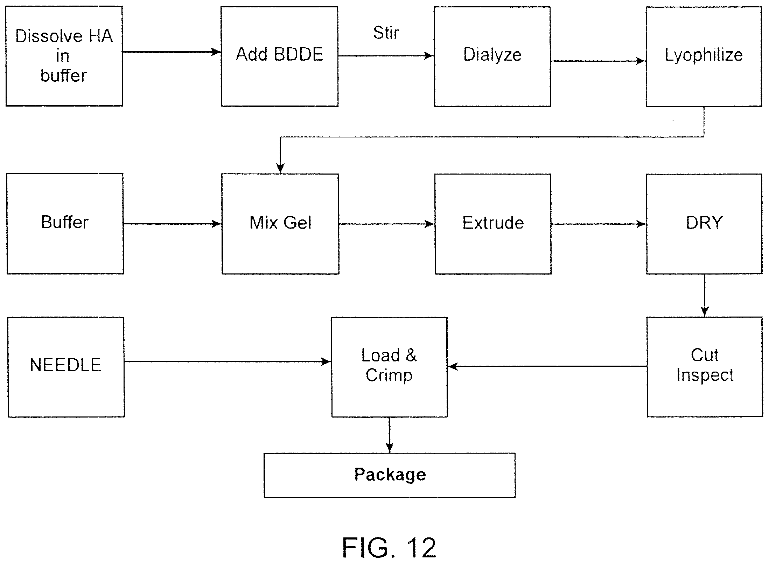

Fig. 12 shows a schematic of preparing the threads in accordance with the methods of the present disclosure.

### DETAILED DESCRIPTION OF THE PREFERRED EMBODIMENTS

### 1. Definitions

Unless defined otherwise, all technical and scientific terms used herein have the same meaning as commonly understood by one of ordinary skill in the art to which this disclosure belongs. As used herein the following terms have the following meanings.

As used herein, the term "comprising" or "comprises" is intended to mean that the compositions and methods include the recited elements, but not excluding others. "Consisting essentially of" when used to define compositions and methods, shall mean excluding other elements of any essential significance to the combination for the stated purpose. Thus, a composition consisting essentially of the elements as defined herein would not exclude other materials or steps that do not materially affect the basic and novel characteristic(s) of the claimed disclosure. "Consisting of" shall mean excluding more than trace elements of other ingredients and substantial method steps. Embodiments defined by each of these transition terms are within the scope of this disclosure.

The term "about" when used before a numerical designation, e.g., temperature, time, amount, and concentration, including range, indicates approximations which may vary by (+) or (-) 10%, 5% or 1%.

As used herein, the term "thread" refers to a long, thin, flexible form of a material. The thread of the disclosure can have a variety of shapes in the cross-section which are discussed below.

The term "hyaluronic acid" or "HA" refers to the polymer having the formula: 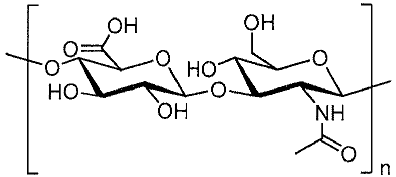 where n is the number of repeating units. All sources of hyaluronic acid are useful in this disclosure, including bacterial and avian sources. Hyaluronic acids useful in this disclosure have a molecular weight of from about 0.5 MDa (mega Dalton) to about 3.0 MDa. In some embodiments, the molecular weight is from about 0.6 MDa to about 2.6 MDa and in yet another embodiment, the molecular weight is from about 1.4 MDa to about 1.6 MDa. The term "modified or activated hyaluronic acid" refers to an HA that is partially cross-linked meaning that that the cross-linking reaction is not fully complete.

The term "ambient conditions" is intended to refer to the typical environmental conditions and preferably, a pressure of about 1 atmosphere and/or temperature of 5 to about 40, and preferably 20 to 30 °C. In some embodiments the ambient conditions comprise a relative humidity of from about 20% to about 80%.

At least a portion of the thread is cross-linked. The term "cross-linked" is intended to refer to two or more polymer chains of hyaluronic acid which have been covalently bonded via a cross-linking agent. Such cross-linking is differentiated from intermolecular or intramolecular dehydration which results in lactone or anhydride formation within a single polymer chain or between two or more chains. Although, it is contemplated that intramolecular cross-linking may also occur in the threads.

"Cross-linking agents" contain at least two reactive groups that create covalent bonds between two or more molecules. The cross-linking agents can be homobifunctional (i.e. have two reactive ends that are identical) or heterobifunctional (i.e. have two different reactive ends). The cross-linking agents should comprise complimentary functional groups to that of hyaluronic acid such that the cross-linking reaction can proceed. In one embodiment, the cross-linking does not form esterified hyaluronic acid. Suitable cross-linking agents include, by way of example only, butanediol diglycidyl ether (BDDE), divinyl sulfone (DVS), and 1-ethyl-3-(3-dimethylaminopropyl) carbodimide hydrochloride (EDC), or a combination thereof. In one embodiment, the cross-linking agent is BDDE.

The term "substantially cross-linked" means that at least about 50% of the cross-linking reaction is complete. In some embodiments, at least about 75% of the cross-linking reaction is complete. In still other embodiments, at least about 80%, about 90%, or even about 100% of the cross-linking means that at least about. In some embodiments, at least about 75% of the cross-linking reaction is complete. In still other embodiments, at least about 80%, about 90%, or even about 100% of the cross-linking reaction is complete. To determine the amount of cross-linking that has occurred a variety of methods including, but not limited to, size exclusion chromatography, light scattering, viscosity, or rheometry, etc., can be used. This is not to say that the HA is 50% cross-linked, rather the percentage is related to the amount of cross-linking possible, which is determined by the amount of HA and the amount of cross-linker.

"Degradation rate" refers to both *in vitro* and *in vivo* degradation. The amount of degradation may be measured in a variety of ways, including by the *in vitro* assay described in Example 12.

The term "percent moisture" is intended to refer to the total percent of water by weight. In one embodiment, the percent hydration is about 30% or less, or alternatively, about 15% or less, or alternatively, about 10% or less. This can typically be measured by Karl Fisher titration.

The term "ultimate tensile strength" is intended to refer to the tensile strength of the thread which has been normalized with respect to cross-sectional area. The term "tensile strength" is intended to refer to the maximum load a thread can withstand without failing when subjected to tension. In one embodiment, it is contemplated that the ultimate tensile strength is sufficient to pull the thread through the skin and manipulate it once in the skin such that the integrity of the thread is not substantially compromised by, for example, breaking or segmenting. It is contemplated that threads preferably have an ultimate tensile strength of about 3 kpsi ("kilopounds per square inch") or greater, or 5 kpsi or greater, or 10 kpsi or greater, or 15 kpsi or greater or 20 kpsi or greater or 50 kpsi or greater or 75 kpsi or greater.

The threads can be made into a variety of shapes. The term "substantially cylindrical" refers to a thread wherein the cross-section of the thread is round. The term "substantially" as used to refer to shapes of the threads means that at least 50% of the thread has the shaped described. The term substantially is also used to encompass threads which have a variety shapes along the length of the thread. For example, a thread could be substantially cylindrical but the ends of the thread may be tapered. The substantially cylindrical threads can be provided when the contact angle of the aqueous mixture and the substrate on which it is extruded have an equilibrium contact angle of greater than about 90 degrees.

The term "substantially D-shaped" refers to a thread wherein the cross-section is D-shaped or substantially semi-circular. The substantially D-shaped threads have one flat side and one substantially round side. The substantially D-shaped threads can be provided when the contact angle of the aqueous mixture and the substrate on which it is extruded have an equilibrium contact angle of about 90 degrees.

The term "substantially ribbon-shaped" refers to a thread wherein the thickness of the thread is less than about 50% of the width of the thread. In some embodiments, the cross-section is substantially rectangular. The ribbon-shaped threads can be provided when the contact angle of the aqueous mixture and the substrate on which it is extruded have an equilibrium contact angle of less than about 90 degrees. Alternatively, the ribbon-shaped threads can be formed by cutting a wet gel to achieve the desired cross-sectional shape. "Ribbon-shaped" may also include shapes that are substantially ellipsoidal. The term "substantially ellipsoidal" refers to a thread wherein the cross-section is substantially oblong or elliptical.

The term "therapeutic agent" can include one or more therapeutic agents. In still other of the above embodiments, the therapeutic agent is an anesthetic, including but not limited to, lidocaine, xylocaine, novocaine, benzocaine, prilocaine, ripivacaine, propofol or combinations thereof. In still other of the above embodiments, the therapeutic agent includes, but is not limited to, epinephrine, ephedrine, aminophylline, theophylline or combinations thereof. In still other of the above embodiments, the therapeutic agent is botulism toxin. In still other of the above embodiments, the therapeutic agent is laminin-511. In still other of the above embodiments, the therapeutic agent is glucosamine, which can be used, for example, in the treatment of regenerative joint disease. In still other of the above embodiments, the therapeutic agent is an antioxidant, including but not limited to, vitamin E or all-trans retinoic acid such as retinol. In still other of the above embodiments, the therapeutic agent includes stem cells. In still other of the above embodiments, the therapeutic agent is insulin, a growth factor such as, for example, NGF (nerve growth factor), BDNF (brain-derived neurotrophic factor), PDGF (platelet-derived growth factor) or Purmorphamine Deferoxamine NGF (nerve growth factor), dexamethasone, ascorbic acid, 5-azacytidine, 4,6-disubstituted pyrrolopyrimidine, cardiogenols, cDNA, DNA, RNAi, BMP-4 (bone morphogenetic protein-4), BMP-2 (bone morphogenetic protein-2), an antibiotic agent such as, for example, ß lactams, quinolones including fluoroquinolones, aminoglycosides or macrolides, an anti-fibrotic agent, including but not limited to, hepatocyte growth factor or Pirfenidone, an anti-scarring agent, such as, for example, anti-TGF-b2 monoclonal antibody (rhAnti-TGF-b2 mAb), a peptide such as, for example, GHK copper binding peptide, a tissue regeneration agent, a steroid, fibronectin, a cytokine, an analgesic such as, for example, Tapentadol HCl, opiates, (e.g., morphine, codone, oxycodone, etc.) an antiseptic, alpha- beta or gamma-interferon, EPO, glucagons, calcitonin, heparin, interleukin-1, interleukin-2, filgrastim, a protein, HGH, luteinizing hormone, atrial natriuretic factor, Factor VIII, Factor IX, or a follicle-stimulating hormone.

The term "lubricity-enhancing agent" is intended to refer to a substance or solution which when contacted with the dry thread, acts to lubricate the dry thread. A lubricity-enhancing agent can comprise, for example, water and/or an alcohol, an aqueous buffer, and may further comprise additional agents such as polyethylene glycol, hyaluronic acid, and/or collagen.

The term "failure load" is intended to refer to the maximum weight which, when applied to the thread, causes the thread to fail. By "failing," it meant that the thread can break or segment or otherwise lose structural integrity. In some embodiments, the failure stress is about 0.1 pounds or 0.22 kilograms or greater.

The term "aqueous composition" or "aqueous mixture" or other compositions of matter including them used herein is intended to refer to an aqueous composition comprising water, hyaluronic acid, and a cross-linking agent, that is useful for use in an extruder to make fibers. In some embodiments, the composition may further comprise a buffer such that that the pH of the solution changes very little with the addition of components of the composition. The pH of the buffered composition is typically from about 7 to about 10. In certain embodiments the pH is about 7. In certain embodiments, the pH is higher at about 9 or about 10. In some embodiments, the pH can be adjusted by adding an appropriate amount of a suitable base, such as Na₂CO₃ or NaOH. In some embodiments, the buffered aqueous composition comprises phosphate buffered saline. In some embodiments, the buffered aqueous composition comprises tris(hydroxymethyl)aminomethane (Tris), which has the formula (HOCH₂)₃CNH₂. In some embodiments, additional solutes are added to adjust the osmolarity and ion concentrations, such as sodium chloride, calcium chloride, and/or potassium chloride.

The term "buffer" is intended to refer to a solution comprising a mixture of a weak acid and its conjugate base or a weak base and its conjugate acid. Buffer solutions include, but are not limited to, 2-amino-2-methyl-1,3-propanediol, 2-amino-2-methyl-1-propanol, L-(+)-tartaric acid, D-(-)-tartaric acid, ACES, ADA, acetic acid, ammonium acetate, ammonium bicarbonate, ammonium citrate, ammonium formate, ammonium oxalate, ammonium phosphate, ammonium sodium phosphate, ammonium sulfate, ammonium tartrate, BES, BICINE, BIS-TRIS, bicarbonate, boric acid, CAPS, CHES, calcium acetate, calcium carbonate, calcium citrate, citrate, citric acid, diethanolamine, EPP, ethylenediaminetetraacetic acid disodium salt, formic acid solution, Gly-Gly-Gly, Gly-Gly, glycine, HEPES, imidazole, lithium acetate, lithium citrate, MES, MOPS, magnesium acetate, magnesium citrate, magnesium formate, magnesium phosphate, oxalic acid, PIPES, phosphate buffered saline, piperazine potassium D-tartrate, potassium acetate, potassium bicarbonate, potassium carbonate, potassium chloride, potassium citrate, potassium formate, potassium oxalate, potassium phosphate, potassium phthalate, potassium sodium tartrate, potassium tetraborate, potassium tetraoxalate dehydrate, propionic acid solution, STE buffer solution, sodium 5,5-diethylbarbiturate, sodium acetate, sodium bicarbonate, sodium bitartrate monohydrate, sodium carbonate, sodium citrate, sodium chloride, sodium formate, sodium oxalate, sodium phosphate, sodium pyrophosphate, sodium tartrate, sodium tetraborate, TAPS, TES, TNT, TRIS-glycine, TRIS-acetate, TRIS buffered saline, TRIS-HCl, TRIS phosphate-EDTA, tricine, triethanolamine, triethylamine, triethylammonium acetate, triethylammonium phosphate, trimethylammonium acetate, trimethylammonium phosphate, Trizma® acetate, Trizma® base, Trizma® carbonate, Trizma® hydrochloride or Trizma® maleate.

The term "aqueous solvent" is intended to refer to a non-toxic, non-immunogenic aqueous composition. The aqueous solvent can be water and/or an alcohol, and may further comprise buffers, salts and other such non-reactive solutes.

The term "contact angle" or "equilibrium contact angle" refers to a measure of a liquid's affinity for a solid and quantifies the degree of a liquid drop's spread when placed on the solid. In the case of the disclosure, the liquid is the aqueous mixture and the rigid or solid surface is the substrate on which the composition is extruded. The contact angle is a measure of the angle that the edge of an ideal drop makes with a flat surface. The lower that the contact angle is, the greater attraction between the surface and the liquid. For example, water spreads almost completely on glass and has a very low contact angle of nearly 0 degrees. Mercury, in contrast, beads up and spreads very little; its contact angle is very large.

### 2. Cross-linked Hyaluronic Acid Compositions

In one aspect, there is provided an aqueous composition comprising hyaluronic acid and a cross-linking agent, wherein the ratio by weight of the hyaluronic acid and the cross-linking agent is from about 1:2 to about 10:1 or about 1:1 to about 8:1. In one embodiment, the ratio is from about 2.5:1 to about 1.5:1. In another embodiment, the ratio is about 2:1.

In another embodiment, the aqueous composition further comprises water, wherein the concentration of the hyaluronic acid is about 0.1 % to about 2%, or 0.1 % to about 1%, or about 0.25% to about 0.75% w/w. In another embodiment, the concentration of the hyaluronic acid is about 0.40% to about 0.55%. In another embodiment, the concentration of the hyaluronic acid is about 8% to about 15%.

In another embodiment, the aqueous composition excludes components having an average molecular weight of up to about 14 kDa to about 100 kDa or up to about 200 kDa. In another embodiment, the aqueous composition excludes components having a molecular weight of up to about 12 kDa. The amount does not include any cross-linker.

In another embodiment, there is provided a lyophilized composition wherein the composition just described is lyophilized. Prior to lyophilizing, the composition may be optionally dialyzed or diafiltered. Typically, this step is done to remove unreacted cross-linker or cross-linking byproducts. The lyophilized composition may be then optionally sterilized by e-beam irradiation providing a sterilized, lyophilized composition. The lyophilized (and optionally sterilized) composition may be formulated by rehydrating. The HA solids may be from about 10% to about 25% based on the total weight in the rehydrated formulation.

Within the aspects and embodiments disclosed here, in one embodiment, the hyaluronic acid comprises hyaluronic acid having an average molecular weight of about 1 MDa to about 3.5 MDa. In another embodiment, the hyaluronic acid comprises hyaluronic acid having an average molecular weight of about 1.2 MDa to about 3 MDa, about 1.4 MDa to about 2.5 MDa, or about 1.6 MDa to about 2 MDa. In still another embodiment, the molecular weight is about 1.7 MDa. A mixture of hyaluronic acids having different molecular weights can also be employed.

Within the aspects and embodiments disclosed here, in one embodiment, the cross-linking agent is selected from the group consisting of butanediol diglycidyl ether (BDDE), divinyl sulfone (DVS), 1-ethyl-3-(3-dimethylaminopropyl) carbodimide hydrochloride (EDC), and combinations thereof. In another embodiment, the cross-linking agent is BDDE. In another embodiment, the cross-linking agent is DVS. In another embodiment, the cross-linking agent is EDC. In another embodiment, the cross linking agent is covalently linked to the hyaluronic acid. In another embodiment, the cross-linking agent is not covalently linked to the hyaluronic acid.

In another aspect, there is provided a sterilized, cross-linked hyaluronic acid having a degradation rate comparable to or faster than the degradation rate of cross-linked hyaluronic acid prior to sterilization.

In another aspect, there is provided a thread comprising a composition comprising hyaluronic acid (e.g., and without limitation, the aqueous compositions, the lyophilized compositions, and the aqueous mixtures) provided herein. In another embodiment, the thread has a failure of stress of 0.1 pounds or greater. In another embodiment, the thread is braided, coiled, layered or woven to form a material. In another embodiment, the thread is substantially cylindrical, substantially D-shaped, substantially ribbon-shaped, or substantially ellipsoidal. In another embodiment, the thread further comprises a member selected from the group consisting of a therapeutic agent, a diagnostic agent, a fibrogenesis-enhancing agent, a lubricity-enhancing agent, a biodegradation impeding agent, and combinations thereof.

In one embodiment, the threads as disclosed herein have an elasticity along their length of less than about 150 percent, or about 100 percent, or about 50 percent, or about 25 percent, or less than about 50 percent. In another embodiment, the cross-linked compositions and threads as disclosed herein are not viscoelastic. In another embodiment, the compositions and threads as disclosed herein are not amorphous thermoplastic biomaterials. In one embodiment, the compositions and threads as disclosed herein do not comprise collagen and/or ortic acid.

### 3. Methods of Making the Threads

In another aspect, there is provided a cross-linked hyaluronic acid thread, prepared by the process of:
a) combining hyaluronic acid and a cross-linking agent in an aqueous composition until the hyaluronic acid is substantially cross-linked to provide a substantially cross-linked composition;
b) extruding the substantially cross-linked composition to provide a wet thread; and
c) drying the wet thread to provide a cross-linked hyaluronic acid thread.

In some embodiments, after combining the HA and cross-linking agent, the composition is lyophilized. It is contemplated that when the BDDE is combined with the HA, the HA is activated, and the cross-linking occurs during the lyophilization when the BDDE is more concentrated. It is further contemplated that cross-linking can continue to occur during the drying step.

In another embodiment, the ratio of hyaluronic acid to cross-linking agent is from about 1:2 to about 10:1 or about 1:1 to about 8:1. In one embodiment, the ratio is from about 2.5:1 to about 1.5:1. In another embodiment, the ratio is about 2:1.

In another embodiment, the method further comprises the step of sterilizing the substantially cross-linked composition prior to extruding. In another embodiment, the method further comprises the step of sterilizing the wet thread prior to drying. In another embodiment, the step of sterilizing comprises e-beam sterilization. It is contemplated that the compositions may also be sterilized by a variety of other methods, including ethylene oxide and autoclave. These methods of sterilization may be employed in all sterilizing steps of the process described herein.

In another embodiment, the method further comprises the step of lyophilizing the composition prior to extruding to provide a lyophilized composition. In another embodiment, the method further comprises the step of dialyzing the composition prior to lyophilizing to provide a dry dialyzed composition.

In another embodiment, the method further comprises the step of dialyzing the composition prior to extruding to provide a dialyzed substantially cross-linked composition. In another embodiment, the method further comprises the step of sterilizing the dry, dialyzed substantially cross-linked composition to provide a sterilized dry dialyzed substantially cross-linked composition. In another embodiment, the step of sterilizing comprises e-beam sterilization or autoclave sterilization or ethylene oxide sterilization.

In another embodiment, the method further comprises the step of hydrating the sterilized dry dialyzed substantially cross-linked composition to provide the substantially cross-linked composition. In another embodiment, the hydrating step comprises a buffer.

In another embodiment, the dialyzed substantially cross-linked composition excludes components having an average molecular weight of less then about 14 kDa, or less than about 12 kDa.

In another embodiment, the drying step comprises from about 36 hours to about 60 hours, or about 48 hours. In another embodiment, the method further comprises the step of applying to the dry hyaluronic acid thread a sufficient amount of a therapeutic agent, a diagnostic agent, a fibrogenesis-enhancing agent, a biodegradation impeding agent, a lubricity-enhancing agent or combinations thereof, optionally followed by the step of re-drying the thread.

In another embodiment, the step of extruding comprises extruding the wet thread onto a substrate. In another embodiment, the substrate is selected from the group consisting of polytetrafluoroethylene (PTFE), expanded PTFE, nylon, polyethylene terephthalate (PET), polystyrene, silicon, polyurethane, and activated cellulose.

In another embodiment, the cross-linking agent is selected from the group consisting of butanediol diglycidyl ether, divinyl sulfone, 1-ethyl-3-(3-dimethylaminopropyl) carbodimide hydrochloride, and combinations thereof. In another embodiment, the cross-linking agent is butanediol diglycidyl ether.

### 4. Methods of Using the Cross-Linked Hyaluronic Acid Threads

In another aspect, there is provided a non-therapeutic method of treating a wrinkle in a patient in need thereof, said method comprising:
1) inserting a thread provided herein into skin of the patient adjacent to or under the wrinkle; and
2) applying the thread adjacent to or under the wrinkle thereby treating the wrinkle.

In another embodiment, the steps 1) and 2) are performed 2 to 6 times. In another embodiment, the thread is inserted by a needle. In another embodiment, the method further comprises removing the needle from the skin. In another embodiment, the method further comprises hydrating the thread. In another embodiment, prior to step 1), a lubricity enhancing agent is applied to the thread.

### Methods of Treating a Wrinkle

It is contemplated that threads have an improved ability to promote fibrogenesis and/or tissue repair *in vivo* by forming a scaffold-like structure in the body for collagen deposition. This tissue repair could prolong the "filler" effects of the thread when used to treat or fill a wrinkle *in vivo* far beyond the half-life of the hyaluronic acid-based thread. This is described in Example 8.

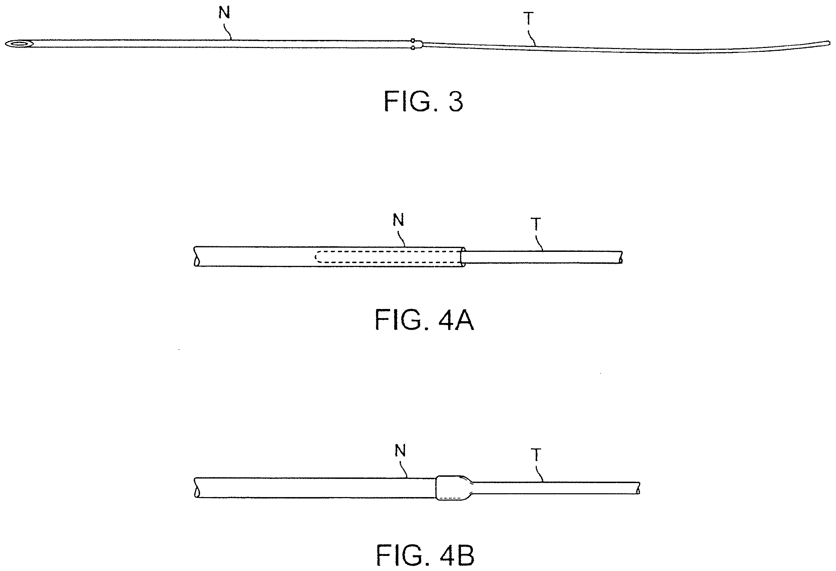

In some embodiments, the present invention is directed to a non-therapeutic method of treating a wrinkle in a patient in need thereof by 1) inserting the thread skin of the patient adjacent to or under the wrinkle; and 2) applying the thread adjacent to or under the wrinkle thereby treating the wrinkle. These steps can be performed at least once and up to 6 times to treat each wrinkle. In some embodiments, the thread is attached to the distal end of a syringe as shown in Figs. 3, 4A and 4B. The thread is inserted by a needle which needle is then removed. Optionally and as necessary, the thread is hydrated with water or saline, or by the fluids normally perfusing the surrounding tissue. Further, the remainder of the wrinkle can be filled with a biocompatible material such as a phase transfer Pluronic™ which can be introduced as a liquid and which solidifies *in vivo.* Alternatively, conventional hyaluronic acid gel can be introduced to fill the wrinkle. In either case, the formed web acts to maintain the biocompatible filler at the site of the wrinkle.

In some embodiments, a non-therapeutic method of treating a wrinkle in a subject is provided. In some embodiments, the attending clinician may numb the treatment area according to procedures known in the art using a variety of anesthetics, including, but not limited to, topical lidocaine, ice or a block with lidocaine injection. For example, the wrinkle may be in the peri-orbital region as illustrated in Fig. 5A. The thread may be attached to a needle as illustrated, for example, in Figs. 3, 4A and 4B. The distal end of the needle may be inserted through the skin surface of the subject into the skin adjacent to or within the wrinkle as illustrated, for example, in Fig. 5B. In some embodiments, the thread is inserted into the subcutaneous space instead of the dermis. The needle then may traverse the dermis or subcutaneous space of the subject beneath the wrinkle as illustrated, for example, in Fig. 5C. The needle then may exit the skin of the subject at the opposite margin of the wrinkle, as illustrated, for example, in Fig. 5D. The needle may then be pulled distally until it is removed from the subject such that the thread is pulled into the location previously occupied by the needle beneath the wrinkle, as illustrated, for example, in Fig. 5E. Finally, excess thread is cut from the needle at the skin surface of the subject which leaves the thread implanted as illustrated, for example, in Fig. 5F.

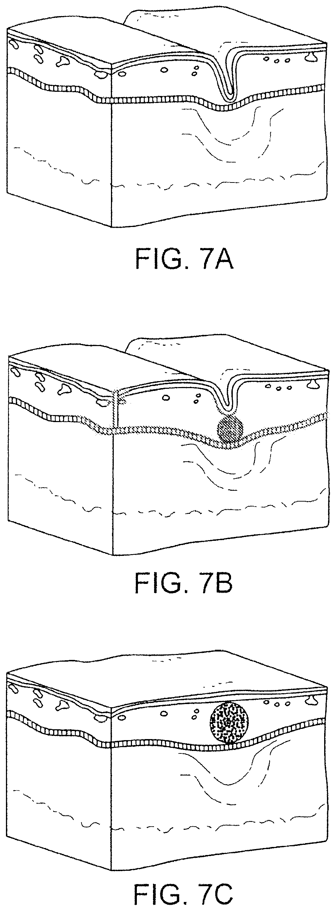

While not wishing to be bound by theory, the method above may successfully treat wrinkles as shown in Figs. 7A, 7B and 7C. A typical wrinkle is illustrated in Fig. 7A. Fig. 7B illustrates a thread implanted beneath a wrinkle that is not yet hydrated. As the thread implanted beneath the wrinkle becomes fully hydrated the surface appearance of the wrinkle is concurrently flattened as illustrated in Fig. 7C.

In some embodiments, the thread is manipulated in such a fashion such that one end of the thread is sufficiently hard such that the thread is used to penetrate the skin. This may be accomplished by coating the thread with a hardening material, such as a sugar coating, In another embodiment, the thread is coated in its entirety, for example with a sugar coating, to provide the thread with increased columnar strength.

### Facial Contouring

It is contemplated that the threads are useful in facial contouring. What is meant by facial contouring is that the threads can be applied to any area of the face, neck, or chest that the patient desires to have augmented, including, by way of example only, the lips, the nasolabial fold, and tear trough.

Lip augmentation is a commonly desired aesthetic procedure. Typically, the aesthetic goal is fuller, plumper lips. Some psychology studies have described an increased attraction by males for females with fuller lips (Lip Size Key to Sexual Attraction, 4 March, 2003. http://news.bbc.co.uk/2/hi/health/2817795.stm). The hypothetical explanation for this phenomenon is that lip fullness or plumpness is correlated with increased estrogen levels and is therefore perceived as a sign of fertility. Areas of enhancement can include the vermillion border (or white roll) for lip effacement and contouring and the wet-dry mucosal junction for increasing fullness. Other techniques include more diffuse infiltration of the orbicularis oris muscle.

Lip contouring and augmentation by temporary soft tissue augmentation products is a popular, low risk option due to the minimal invasiveness and temporary nature of the procedure. The major shortcomings of soft tissue augmentation products currently used in lip procedures are that it is (a) painful, (b) difficult to consistently and homogenously inject the gel into the desired location, and (c) the gel can migrate over the lifetime of the implant causing the aesthetic results to change.

The present disclosure addresses the shortcomings described above. Beyond addressing the above-listed shortcomings for existing temporary soft tissue augmentation products described above, it has been found that the HA thread-based method of enhancing lip appearance is very quick. A typical patient may have 3 threads in their lip(s) in only 3 minutes. Current soft tissue augmentation product lip procedures can take 15 to 20 minutes.

In embodiments, directed to facial contouring, the attending clinician may numb the treatment area according to procedures known in the art using a variety of anesthetics, including, but not limited to, topical lidocaine, ice or a block with lidocaine injection. Threads made of HA (hyaluronic acid) can be attached to the proximal end of a needle and pulled into the lip. The needle can serve as a precise guide, and also be used to predict and correct the implant location prior to pulling the thread into the desired location. This precise delivery mechanism can be used to deliver threads along the vermillion border for contouring, superficially if desired, as well as at the wet-dry junction for plumping, deeper into the lip if desired.

It is contemplated that when the thread is used for facial contouring, any number of threads may be used depending on the desired effect and the size of the thread. For example, description of the procedure done for the lip augmentation and contouring is discussed below in Example 11.

It is has been surprisingly and unexpectedly found that that threads may be implanted in various tissue planes of the patient to provide a more natural look when performing facial contouring. For example, the threads may be implanted in a manner that forms a hammock in the desired location. Given the unique properties of the threads, the attending clinician may deposit or implant the threads in the epidermis, the dermis, and the subcutaneous layer.

This technique can is enabled by the precision with which the threads can be placed, and their size relative to the dermis and underlying structures. Threads can impart different effects on facial features such as wrinkles, contours, folds and troughs depending on where they are implanted.

For example, recent clinical experience indicates that placing a thread (in this case one that was approximately .008" in diameter) deeply, for example in the subcutaneous space, along the axis of a forehead wrinkle can help soften then appearance of the wrinkle that forms when the patient animates, by flexing their forehead - which would typically exacerbate the appearance of the wrinkle. These types of dynamic wrinkles are currently only well treated with Botox®, which has the undesirable effect of preventing the patient from expressing all facial expressions. Further, recent clinical experience shows that static wrinkles, ones that are visible in repose, can be effectively treated by placement of a thread (from .004 to .008" in diameter) superficially, for example within the dermis.

The technique of stratifying the thread implant tissue planes is also successfully used in improving the appearance of nasolabial folds (up to 4x .008" threads), glabellar lines, marionette lines, and lips.

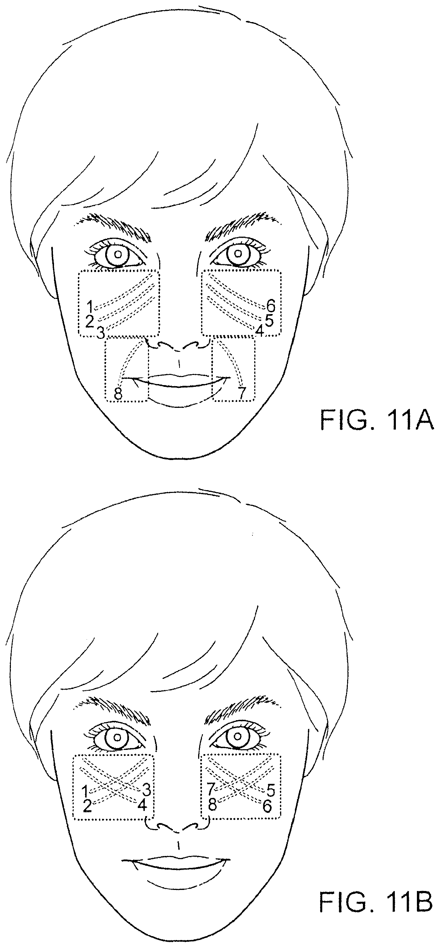

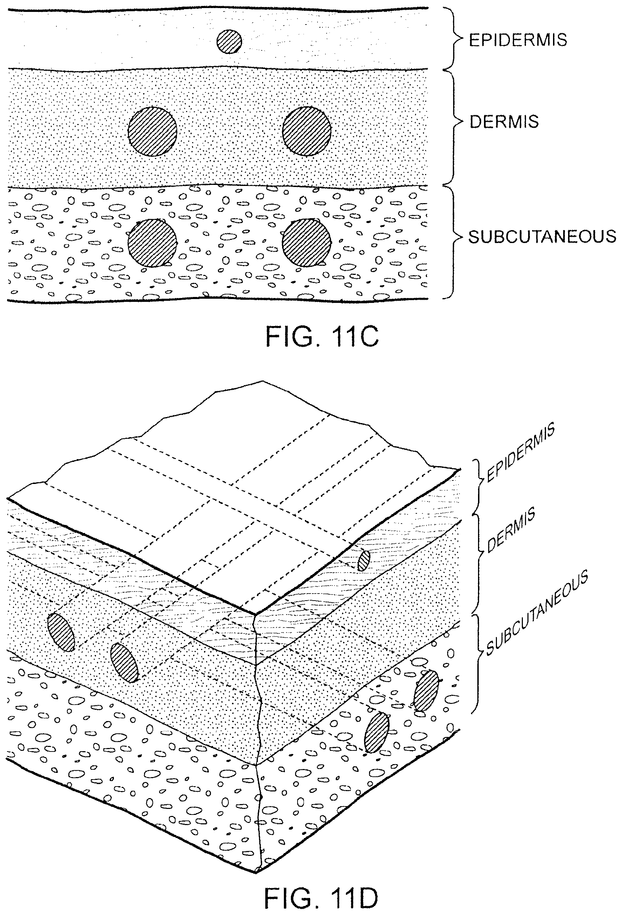

This is another technique that is enabled by the HA threads and their implantation method. To smooth the appearance of hollows or troughs such as the tear trough, or otherwise contour the face in areas such as the cheek bones, chin, for example, threads can be implanted in hatch (see, Fig. 11A) and or cross-hatched patterns (see, Fig. 11B) to effect areas greater than the width of a single thread. As seen in Fig. 11A and 11B, two patients have their tear troughs effectively smoothed out by placing threads parallel in one case (Fig. 11A) and cross-hatched in another case (Fig. 11B). The cross-hatching could be done obliquely to the initial direction, as was the case in Fig. 11B, or perpendicularly. Further, the hatches can be at different tissue planes too.

In another embodiment of this technique, the hatching can be done obliquely to the directionality of the area being treated. For example, in Fig. 11A below the threads are placed aligned to the axis of the tear trough. Instead, the threads could be placed obliquely to the axis of the tear trough to support the tissue in the area differently.

It is contemplated that implanting the threads in various planes may also be done in the treatment of wrinkles as described above.

### 5. Kits

In another aspect, there is provided a kit of parts comprising a thread and a means for delivery of the thread to a patient. In another embodiment, the means for delivery to a patient is a syringe, a needle, or an air gun. In another embodiment, the kit of parts is for use in treating a wrinkle in a patent.

The size (or diameter) of the needle may depend on the use of the thread, and therefore also be based on the cross-sectional area of the thread used. The outer diameter of the needle or syringe may be greater than or equal to the cross-sectional area of the thread used to lessen the tensile requirement of the thread as it is being applied to the skin. It is further contemplated that the outer diameter of the thread may be larger than the outer diameter of the needle. Skin is quite pliable so by having a smaller diameter needle can allow the puncture size to be small even with the use of a larger diameter thread. Further, the thickness of the thread would be different in the case where the thread is a suture in comparison to the treatment of fine lines and wrinkles where it may be that a thinner thread is used. More than one thread may also be attached to a single needle.

Further, the size of the delivery device, a needle, will be dependent on its intended use and the size of the thread. It is contemplated that for use in facial contouring and or wrinkle filling a 0.006 to about 0.008" diameter thread or a 0.003 to about 0.004" diameter thread will be sufficient. In one embodiment, the needle is stainless steel. In other embodiments, the size of the thread is from about 0.01" to 0.02" in diameter.

The thread attachment to the needle can be either a mechanical attachment and/or with the use of an adhesive, such as cyanoacrylate. In one embodiment, the thread woven or looped through holes in the proximal end of the needle, or alternatively, the thread wrapped around the proximal end of the needle, or alternatively, the thread threaded thru an eyelet of the needle and either tied or bonded with an adhesive to form a loop, or alternatively, the thread secured (either mechanically or bonded with an adhesive) within a hole in the proximal end of the needle. In another embodiment, the thread can be made to form a physical attachment to the needle during the drying process as the thread forms from the gel. For example, if a needle is used which has pores in the proximal end, the pores can fill with the gel during the extrusion process and the thread would be thus be secured upon drying. The needle can be rigid or flexible to enable the user to track the needle under the wrinkle within the skin. Further, the needle may be equipped with a ramp to guide the needle at a desired depth within the skin, and after needle insertion, the guide may be unclasped as the needle is brought through the skin surface. In some embodiments, the thread is attached to a needle.

It is further contemplated that the kit comprises a needle and the thread attached thereto, is packaged sterile, and intended for single use. Alternatively, a kit can comprise several needles, each with an attached thread. In an additional embodiment, a kit includes threads of different sizes to enable treatment options for the physician while minimizing the number of required needle sticks. In yet another embodiment, the kit includes threads and needles of different length and curved shapes to simplify implantation in areas that are difficult to access or treat with a straight needle, for example near the nose, around the eyes and the middle portion of the upper lip.

### Examples

The present disclosure is further defined by reference to the following examples. It will be apparent to those skilled in the art that many modifications, both to threads and methods, may be practiced without departing from the scope of the current disclosure. The hyaluronic acid and cross-linking agents are available from commercial sources.

### Example 1: General Synthesis of Cross-Linked Threads

In one embodiment, the thread fabrication process is as described below:
1. Prepare a solution of hyaluronic acid - about 0.25 to about 0.75% w/w.. It is contemplated that the HA molecular weight may range from about 750 KDa to about 3 MDa. The pH of the solution may range from about 6.0 to about 9.0.
2. Add Butanediol diglycidyl ether (BDDE) in a ratio of BDDE to HA ranging from 2:1 to 0.25:1 and stir the solution for about 6 to about 48 hours. Other crosslinkers commonly used to cross-link hyaluronic acid such as divinyl sulfone may also be used. Based on the cross-linker, the pH of the solution and the concentration of the cross-linker may also be varied. Also, additional ratios of HA and BDDE may also be employed. BDDE and HA are allowed to react for any where from about 6 hours to about 24 hours or longer.
3. If the level of unreacted cross-linker or cross-linking byproducts is high, dialyze or diafilter the cross-linked hyaluronic acid solution.
4. Lyophilize the dialyzed cross-linked HA solution.
5. The lyophilized cross-linked HA may be sterilized by e-beam irradiation. Other forms of sterilization like gamma irradiation, ethylene oxide, steam etc. may also be used.
6. Hydrate the lyophilized cross-linked HA. The pH of the hydration solution may range from about 6.0 to about 9.0. The solids content of the formulation may range from about 10 to about 25%.
7. Extrude threads using nozzles ranging from 15G to 20G.
8. Dry the extruded threads at ambient temperature for about 6 to about 72 hrs.

### Example 2: Synthesis of Cross-Linked Threads

### Example 2A

A 0.5% w/w hyaluronic acid (HA) (about 1.5 MDa) solution was prepared by dissolving the HA in 10 mM TRIS buffer (pH 7.00). Butanediol diglycidyl ether (BDDE) was added to the HA solution and the solution was stirred overnight. The ratio of BDDE to HA was 2:1. The substantially cross-linked HA solution was then dialyzed against excess deionized water using a dialysis membrane with a molecular weight cut-off of about 12 to about 14 KDa. The dialyzed solution was then lyophilized to obtain dry substantially cross-linked hyaluronic acid.

The dry cross-linked hyaluronic acid (2.0 g) was exposed to 25 KGy e-beam (Irradiation temperature 1 - 3 °C), and 1.0 g of dry cross-linked hyaluronic acid was not exposed to irradiation. Both groups were then formulated to 16% solids (w/w) in 10 mM TRIS buffer (pH 7.00).

The gel with irradiated cross-linked HA was then extruded through two different sized nozzles (20G and 16G). The extruded threads were dried for about 48 hrs at ambient temperature. The nominal dimensions of the dry threads extruded with the 20G nozzle were of a 0.007 inch thickness and 0.011 inch width.

The nominal dimensions of the dry threads extruded with the 16G nozzle were 0.016 inch thickness and 0.019 inch width.

The gel with non-irradiated cross-linked HA was then extruded through a 16G nozzle. The extruded threads were dried for about 48 hours at ambient temperature. The nominal dimensions of the dry threads were 0.025 inch thickness and 0.029 inch width.

### Example 2B

A 0.5% w/w hyaluronic acid (HA) (about 2.7 MDa) solution was prepared by dissolving the HA in 10 mM TRIS buffer (pH 7.00). Butanediol diglycidyl ether (BDDE) was added to the HA solution and the solution was stirred overnight. The ratio of BDDE to HA was 2:1. The cross-linked HA solution was then dialyzed against excess deionized water using a dialysis membrane with a molecular weight cut-off of 12 - 14 KDa. The dialyzed solution was then lyophilized to obtain dry substantially cross-linked hyaluronic acid.

The dry substantially cross-linked hyaluronic acid (2.0 g) was exposed to 25 KGy e-beam (Irradiation temperature 1 - 3 °C), and 1.0 g of the dry substantially cross-linked hyaluronic acid was not exposed to e-beam irradiation. Both groups were then formulated to 16% solids (w/w) in 10 mM TRIS buffer (pH 7.00).

The gel with irradiated cross-linked HA was then extruded through two different sized nozzles (20G and 16G). The extruded threads were dried for about 48 hours at ambient temperature. The nominal dimensions of the dry threads extruded with the 20G nozzle were 0.009 inch thickness and 0.010 inch width. The nominal dimensions of the dry threads extruded with the 16G nozzle were not measured.

The gel with non-irradiated cross-linked HA was then extruded through a 16G nozzle. The extruded threads were dried for about 48 hours at ambient temperature. The nominal dimensions of the dry threads were 0.026 inch thickness and 0.016 inch width. Threads fabricated as described above were then placed in a physiological buffer (PBS) and incubated at 37 °C. All the threads substantially retained their structure for at least 3 days.

### Example 3: Washing (Re-Hydrating) and Re-Drying the Thread

In one embodiment, the threads are washed. The dry threads are washed with an aqueous solvent to remove contaminants. The washing is performed by various methods, such as submersion in an aqueous solvent or by using a concurrent flow system by placing the thread in a trough at an incline and allowing an aqueous solvent to flow over the thread. In addition, the thread, once it is rehydrated, is stretched prior to re-dying. The rehydrated and washed thread is then re-dried to provide the dry thread. The re-drying is typically performed under ambient conditions (i.e. ambient temperature and/or pressure) for from about 8 hours to about 24 hours or until the dry thread has a percent moisture of less than about 30%. The structural integrity of the thread, the increase in the overall length of the thread, is observed upon washing several times (e.g. 10 or more times).

### Example 4: Comparison of Ultimate Tensile Strength of Different Threads

Threads prepared as described herein are tested for tensile strength using a force gauge (e.g. Digital Force Gauge by Precision Instruments). Monocryl® is used as purchased as a standard. Failure is determined by force at which the thread broke. A zero measurement is the result of an inability to form a thread of testing quality.

### Example 5: Treatment of Wrinkles of a Cadaver with Hyaluronic Acid Threads

Hypodermic needles (22 Ga) are affixed with single or double strands of hyaluronic acid threads (cross-linked with BDDE) with LocTite 4014. More than one thread is used to treat the wrinkles in order to achieve the desired fill effect (two to four threads). Since cadaveric tissue does not have the same hydration characteristics as living tissue, the threads are hydrated by applying a 0.9% saline solution to the treated area. The effect of the thread hydration upon the lessening of the wrinkle is observed visibly.

### Example 6: Placement of Hyaluronic Acid Threads in Dogs

Acute and chronic canine studies are performed. Hypodermic needles (22 to 25 Ga) are affixed with single or double strands of hyaluronic acid threads (cross-linked with BDDE), ranging from thicknesses of 0.004 in to 0.008 in. The samples are e-beam sterilized at 29 kGy. The impact on the skin surface of the animals, e.g., in the form of a linear bump is visibly observed. The threads are further observed by dissection (e.g., after 3 days).

### Example 7:: Organization of the Threads via Atomic Force Microscopy (AFM)

The organization of the cross-linked threads and gels is determined by atomic force microscopy (AFM). The AFM images are collected using a NanoScope III Dimension 5000 (Digital Instruments, Santa Barbara, California, USA). The instrument is calibrated against a NIST traceable standard. NanoProbe® silicon tips are used. Image processing procedures involving auto-flattening, plane fitting or convolution are employed. One 20 cm x 20 cm area is imaged at a random location for both the gel and the thread samples. The topography differences of these images are presented in degree of shading where the dark areas are low and the light areas are high.

### Example 8: In Vitro or In Vivo Testing Regarding Increase in Fibrogenesis

The *in vivo* stimulation of collagen production caused by the threads of the disclosure is accomplished using methods known in the art. For example, according to the methods of Wang et al. (Arch Dermatol. (2007) 143(2):155-163), the thread is applied to a patient followed by a biopsy of the treatment area at one or more time intervals following treatment. The *de novo* synthesis of collagen can then be assessed using immunohistochemical analysis, quantitative polymerase chain reaction, and electron microscopy.

It is contemplated that the threads as disclosed herein will result in the synthesis of collagen at the treatment site, thus prolonging the wrinkle filling effects of the threads beyond the half-life the thread.

### Example 9: Water Content of Dry Threads by Karl Fisher Titration

Hyaluronic acid (HA) is a water binding polymer that is present in the mammalian tissues. The swelling and water intake within HA aggregates depend on propensity of water molecules to interact with the polar groups of this polymer. IR spectroscopy studies on HA films in the dried and hydrated states have demonstrated that the presence of intramolecular hydrogen-bonded organization in the dried state (Haxaire et al. (2003) Biopolymers, 72(3):149-161). Upon interaction with water, this organization develops into hydrogen-bonded intermolecular structures where nano aggregates of water bridge the HA molecules. Intrachain hydrogen-bonded structure that exists in the dried states contain N-H···(-)O-C=O pairs. At higher humidity, N-H and (-)O-C=O groups are hydrated with nanodroplets containing 25 water molecules.

Threads made by the methods above are tested for the percent moisture via Karl Fisher titration. One water molecule per disaccharide unit will give 4.5% of water content in the HA preparation.

### Example 10: Organization of the Threads via Transmission Electron Microscopy (TEM)

Samples of hyaluronic acid gel and thread as prepared in Example 1 are removed from refrigerator then capped with protective carbon, iridium metal, and local platinum. TEM-ready samples is prepared by focused ion beam (FIB) milling. The fiber samples are cross-sectioned in the longitudinal direction using the *in situ* FIB lift out method with a FEI 830 Dual Beam FIB fitted with an Omniprobe Autoprobe 2000. The gel sample is a random cut. TEM imaging is typically performed at room temperature in bright-field TEM mode using a FEI Tecnai TF-20 operated at 200kV.

### Example 11: Lip Augmentation

A patient is implanted with HA threads for lip enhancement, either contouring and/or plumping. The patient can receive only topical anesthetic on the face, but it is generally not applied specifically to the lips. The following procedure is followed:
- Peal open the pouch and remove the sterile tray holding the HA (hyaluronic acid) threads.
- Using sterile gloves or a sterile implement such as forceps, remove the desired HA thread from the tray.
- Insert the sharp end of the needle into one margin of the intended treatment area.
- Translate the needle within the skin under or near the intended treatment area. If the needle is not in a desired location at any point, gently retract the needle and reinsert to correct the location.
- Exit the skin at the opposing margin of the intended treatment area using the sharp end of the needle. If the needle is not in the desired location, gently retract the needle and reinsert to correct the location.
- Upon confirming the desirable location of the needle, swiftly pull the needle distally, pulling the thread into place within the skin.
- Using sterile surgical scissors or scalpel, cut the excess thread protruding from the skin on both margins of the treatment area. This effectively separates the needle, which should be discarded appropriately.

Areas of enhancement include the vermillion border (or white roll) for lip effacement and contouring, the wet-dry mucosal junction for increasing fullness. Other techniques include more diffuse infiltration of the orbicularis oris muscle. The attending clinician is able to select the location of the thread placement, the number of threads and the size of the threads depending on desired effect. It is contemplated that each area is treated with 1 to 2 threads wherein each thread has a diameter of anywhere from 200 microns to about 500 microns when the thread is dry. After hydration, it is contemplated that the thread is from 0.5 millimeters to about 5 millimeters.

### Example 12: Test method for characterizing the degradation profile of cross-linked threads

The following describes the in vitro method for characterizing the degradation profile of cross-linked hyaluronic acid (XL HA) threads of the disclosure. The following procedure is used.
- If not already available, prepare 0.01 M, pH 7.4 PBS solution and store refrigerated.
- Obtain plastic 2 mL microcentrifuge tubes.
- Label tube caps with assigned sample ID numbers. Samples are typically run in duplicate, therefore a -1, -2 suffix may be used to identify replicates. ID numbers will be used to track degradation data in a data tracking spreadsheet.
- Place tubes into microcentrifuge tube rack.
- Pipette 1 ml of PBS (phosphate buffered saline) solution into each tube.
- Cut a piece (or pieces) of the test sample thread into 4 sections approximately 0.75" long.
- Place the sections of thread into one tube and close the cap.
- Repeat until all samples are in the tubes.
- Place the samples into an incubator set at 37 °C.
- Assess degradation profile by scoring sample state using the scoring system defined below. Assess samples on a daily (work week) basis unless otherwise specified in an experimental plan.
- The deg study scoring system ranges from 0 to 3 according to the following guideline.

| **Score** | **Description** |
|---|---|
| 3 | 90 - 100% of thread still present in vial |
| 2 | 50 - 90% of thread still present in vial |
| 1 | 10 - 50% of thread still present in vial |
| 0 | 0 - 10% of thread still present in vial |

## Claims

1. A cross-linked hyaluronic acid thread, obtainable by the process comprising the following steps:
a) combining hyaluronic acid and a cross-linking agent in an aqueous composition until the hyaluronic acid is substantially cross-linked to provide a substantially cross-linked composition;
b) extruding the substantially cross-linked composition to provide a wet thread; and
c) drying the wet thread to provide the cross-linked hyaluronic acid thread.

2. The cross-linked hyaluronic acid thread of claim 1, wherein the ratio of hyaluronic acid to the cross-linking agent is from 1:2 to 10:1.

3. The cross-linked hyaluronic acid thread of claim 1 or 2, wherein the cross-linking agent is selected from the group consisting of butanediol diglycidyl ether, divinyl sulfone, 1-ethyl-3-(3-dimethylaminopropyl)carbodimide hydrochloride, and combinations thereof.

4. The cross-linked hyaluronic acid thread of any of claims 1 to 3, wherein the process further comprises the step of sterilizing the substantially cross-linked composition prior to extruding.

5. The cross-linked hyaluronic acid thread of any of claims 1 to 4, wherein the process further comprises the step of sterilizing the wet thread prior to drying.

6. The cross-linked hyaluronic acid thread of any of claims 1 to 5, wherein the process further comprises the step of lyophilizing the cross-linked composition prior to extruding to provide a lyophilized substantially cross-linked composition.

7. The cross-linked hyaluronic acid thread of any of claims 1 to 6, wherein the process further comprises the step of dialyzing the cross-linked composition prior to extruding to provide a dialyzed substantially cross-linked composition.

8. The cross-linked hyaluronic acid thread of any of claims 1 to 7, wherein the process further comprises the step of dialyzing the cross-linked composition prior to lyophilizing to provide a dry dialyzed substantially cross-linked composition.

9. The cross-linked hyaluronic acid thread of any of claims 1 to 8, wherein the process further comprises the step of applying to the dry hyaluronic acid thread a sufficient amount of a therapeutic agent, a diagnostic agent, a fibrogenesis-enhancing agent, a lubricity-enhancing agent, a biodegradation impeding agent, or combinations thereof, optionally followed by the step of re-drying the thread.

10. A kit of parts comprising the thread of any of claims 1 to 9 and a means for delivery of the thread to a patient.

11. The kit of parts of claim 10, wherein the means for delivery to a patient is selected from a syringe, a needle, or an air gun.

12. A non-therapeutic method of providing facial contouring in a patient in need thereof, said method comprising the following steps:
1) inserting the cross-linked hyaluronic acid thread of any of claims 1 to 9 into skin of the patient adjacent to or under a treatment location; and
2) applying the cross-linked hyaluronic acid thread adjacent to or under the treatment location thereby providing facial contouring.

13. The non-therapeutic method of claim 12, wherein the treatment location is selected from the lips, the nasolabial fold, and the tear trough.

14. A cross-linked hyaluronic acid thread of any of claims 1 to 9 for use in a method for providing facial contouring in a patient in need thereof, said method comprising the following steps:
1) inserting the cross-linked hyaluronic acid thread of any of claims 1 to 9 into skin of the patient adjacent to or under a treatment location; and
2) applying the cross-linked hyaluronic acid thread adjacent to or under the treatment location thereby providing facial contouring.

15. The cross-linked hyaluronic acid thread of any of claims 1 to 9 for use according to claim 14, wherein the treatment location is selected from the lips, the nasolabial fold, and the tear trough.

## Patentansprüche

1. Vernetzter Hyaluronsäurefaden, erhältlich durch das Verfahren, das die folgenden Schritte umfasst:
a) Zusammenführen von Hyaluronsäure und einem Vernetzungsmittel in einer wässrigen Zusammensetzung, bis die Hyaluronsäure im Wesentlichen vernetzt ist, so dass eine im Wesentlichen vernetzte Zusammensetzung bereitgestellt wird;
b) Extrudieren der im Wesentlichen vernetzten Zusammensetzung, so dass ein feuchter Faden bereitgestellt wird; und
c) Trocknen des feuchten Fadens, so dass der vernetzte Hyaluronsäurefaden bereitgestellt wird.

2. Vernetzter Hyaluronsäurefaden gemäß Anspruch 1, wobei das Verhältnis von Hyaluronsäure zu dem Vernetzungsmittel von 1:2 bis 10:1 beträgt.

3. Vernetzter Hyaluronsäurefaden gemäß Anspruch 1 oder 2, wobei das Vernetzungsmittel ausgewählt ist aus der Gruppe, bestehend aus Butandioldiglycidylether, Divinylsulfon, 1-Ethyl-3-(3-dimethylaminopropyl)-carbodiimid-Hydrochlorid und Kombinationen davon.

4. Vernetzter Hyaluronsäurefaden gemäß einem der Ansprüche 1 bis 3, wobei das Verfahren ferner den Schritt umfasst, bei dem die im Wesentlichen vernetzte Zusammensetzung vor dem Extrudieren sterilisiert wird.

5. Vernetzter Hyaluronsäurefaden gemäß einem der Ansprüche 1 bis 4, wobei das Verfahren ferner den Schritt umfasst, bei dem der feuchte Faden vor dem Trocknen sterilisiert wird.

6. Vernetzter Hyaluronsäurefaden gemäß einem der Ansprüche 1 bis 5, wobei das Verfahren ferner den Schritt umfasst, bei dem die vernetzte Zusammensetzung vor dem Extrudieren lyophilisiert wird, so dass eine lyophilisierte im Wesentlichen vernetzte Zusammensetzung bereitgestellt wird.

7. Vernetzter Hyaluronsäurefaden gemäß einem der Ansprüche 1 bis 6, wobei das Verfahren ferner den Schritt umfasst, bei dem die vernetzte Zusammensetzung vor dem Extrudieren dialysiert wird, so dass eine dialysierte im Wesentlichen vernetzte Zusammensetzung bereitgestellt wird.

8. Vernetzter Hyaluronsäurefaden gemäß einem der Ansprüche 1 bis 7, wobei das Verfahren ferner den Schritt umfasst, bei dem die vernetzte Zusammensetzung vor dem Lyophilisieren dialysiert wird, so dass eine trockene dialysierte im Wesentlichen vernetzte Zusammensetzung bereitgestellt wird.

9. Vernetzter Hyaluronsäurefaden gemäß einem der Ansprüche 1 bis 8, wobei das Verfahren ferner den Schritt umfasst, bei dem eine ausreichende Menge eines therapeutischen Wirkstoffs, eines diagnostischen Wirkstoffs, eines die Fibrogenese fördernden Wirkstoffs, eines die Schmierfähigkeit verbessernden Wirkstoffs, eines den biologischen Abbau hemmenden Wirkstoffs oder Kombinationen davon auf den trockenen Hyaluronsäurefaden appliziert werden, gegebenenfalls gefolgt durch den Schritt, bei dem der Faden erneut getrocknet wird.

10. Kit aus Teilen, umfassend den Faden gemäß einem der Ansprüche 1 bis 9 und ein Mittel zur Abgabe des Fadens an einen Patienten.

11. Kit gemäß Anspruch 10, wobei das Mittel zur Abgabe an einen Patienten/eine Patientin ausgewählt ist aus einer Spritze, einer Nadel oder einer Luftpistole.

12. Nicht-therapeutisches Verfahren zur Bereitstellung von Gewichtskonturierung bei einem Patienten/einer Patientin, der/die dessen bedarf, wobei das Verfahren die folgenden Schritte umfasst:
1) Einführen des vernetzten Hyaluronsäurefadens gemäß einem der Ansprüche 1 bis 9 in die Haut des Patienten/der Patientin angrenzend an oder unterhalb einer Behandlungsstelle; und
2) Applizieren des vernetzten Hyaluronsäurefadens angrenzend an oder unterhalb der Behandlungsstelle, wodurch Gesichtskonturierung bereitgestellt wird.

13. Nicht-therapeutisches Verfahren gemäß Anspruch 12, wobei die Behandlungsstelle aus den Lippen, der nasolabialen Falte und der Tränenrinne ausgewählt ist.

14. Vernetzter Hyaluronsäurefaden gemäß einem der Ansprüche 1 bis 9 zur Verwendung bei einem Verfahren zur Bereitstellung von Gesichtskonturierung bei einem Patienten/einer Patientin, der/die dessen bedarf, wobei das Verfahren die folgenden Schritte umfasst:
1) Einführen des vernetzten Hyaluronsäurefadens gemäß einem der Ansprüche 1 bis 9 in die Haut des Patienten/der Patientin angrenzend an oder unterhalb einer Behandlungsstelle; und
2) Applizieren des vernetzten Hyaluronsäurefadens angrenzend an oder unterhalb der Behandlungsstelle, wodurch Gesichtskonturierung bereitgestellt wird.

15. Vernetzter Hyaluronsäurefaden gemäß einem der Ansprüche 1 bis 9 zur Verwendung gemäß Anspruch 14, wobei die Behandlungsstelle aus den Lippen, der nasolabialen Falte und der Tränenrinne ausgewählt ist.

## Revendications

1. Fil d'acide hyaluronique réticulé, pouvant être obtenu par le procédé comprenant les étapes suivantes :
a) combiner de l'acide hyaluronique et un agent de réticulation dans une composition aqueuse jusqu'à ce que l'acide hyaluronique soit sensiblement réticulé pour fournir une composition sensiblement réticulée;
b) extruder la composition sensiblement réticulée pour fournir un fil humide; et
c) sécher le fil humide pour fournir le fil d'acide hyaluronique réticulé.

2. Fil d'acide hyaluronique réticulé selon la revendication 1, dans lequel le ratio de l'acide hyaluronique par rapport à l'agent de réticulation est de 1:2 à 10:1.

3. Fil d'acide hyaluronique réticulé selon la revendication 1 ou 2, dans lequel l'agent de réticulation est choisi dans le groupe constitué par l'éther de butanediol de diglycidyle, une sulfone de divinyle, le chlorhydrate de 1-éthyl-3-(3-diméthylaminopropyl)carbodiimide, et des combinaisons de ceux-ci.

4. Fil d'acide hyaluronique réticulé selon l'une quelconque des revendications 1 à 3, dans lequel le procédé comprend en outre l'étape de stérilisation de la composition sensiblement réticulée avant l'extrusion.

5. Fil d'acide hyaluronique réticulé selon l'une quelconque des revendications 1 à 4, dans lequel le procédé comprend en outre l'étape de stérilisation du fil humide avant le séchage.

6. Fil d'acide hyaluronique réticulé selon l'une quelconque des revendications 1 à 5, dans lequel le procédé comprend en outre l'étape de lyophilisation de la composition réticulée avant l'extrusion pour fournir une composition lyophilisée sensiblement réticulée.

7. Fil d'acide hyaluronique réticulé selon l'une quelconque des revendications 1 à 6, dans lequel le procédé comprend en outre l'étape de dialyse de la composition réticulée avant l'extrusion pour fournir une composition sensiblement réticulée dialysée.

8. Fil d'acide hyaluronique réticulé selon l'une quelconque des revendications 1 à 7, dans lequel le procédé comprend en outre l'étape de dialyse de la composition réticulée avant la lyophilisation pour fournir une composition sensiblement réticulée dialysée sèche.

9. Fil d'acide hyaluronique réticulé selon l'une quelconque des revendications 1 à 8, dans lequel le procédé comprend en outre l'étape d'application sur le fil d'acide hyaluronique sec d'une quantité suffisante d'un agent thérapeutique, d'un agent de diagnostic, d'un agent améliorant la fibrogénèse, d'un agent améliorant la lubrification, d'un agent empêchant la biodégradation, ou de combinaisons de ceux-ci, éventuellement suivie de l'étape de séchage à nouveau du fil.

10. Kit de pièces comprenant le fil selon l'une quelconque des revendications 1 à 9 et un moyen pour délivrer le fil à un patient.

11. Kit de pièces selon la revendication 10, dans lequel le moyen de délivrance à un patient est choisi parmi une seringue, une aiguille ou un pistolet à air comprimé.

12. Procédé non thérapeutique pour fournir un remodelage facial chez un patient en ayant besoin, ledit procédé comprenant les étapes suivantes :
1) insertion du fil d'acide hyaluronique réticulé selon l'une quelconque des revendications 1 à 9 dans la peau du patient en un endroit adjacent à ou sous un emplacement de traitement ; et
2) application du fil d'acide hyaluronique réticulé en un endroit adjacent à ou sous l'emplacement de traitement, ce qui permet de réaliser un remodelage facial.

13. Procédé non thérapeutique selon la revendication 12, dans lequel l'emplacement du traitement est choisi parmi les lèvres, le pli nasolabial et la vallée des larmes.

14. Fil d'acide hyaluronique réticulé selon l'une quelconque des revendications 1 à 9, destiné à être utilisé dans un procédé pour fournir un remodelage facial à patient en ayant besoin, ledit procédé comprenant les étapes suivantes :
1) l'insertion du fil d'acide hyaluronique réticulé selon l'une quelconque des revendications 1 à 9 dans la peau du patient en un endroit adjacent à ou sous un emplacement de traitement ; et
2) l'application du fil d'acide hyaluronique réticulé en un endroit adjacent à ou sous l'emplacement de traitement, ce qui permet de réaliser un remodelage facial.

15. Fil d'acide hyaluronique réticulé selon l'une quelconque des revendications 1 à 9 pour une utilisation selon la revendication 14, dans lequel l'emplacement de traitement est choisi parmi les lèvres, le pli nasolabial et la vallée des larmes.
